# EUROPÄISCHE PATENTANMELDUNG

(11) **EP 4 679 443 A1**
(43) Veröffentlichungstag der Anmeldung: **14.01.2026**
(21) Anmeldenummer: 24188373.5
(22) Anmeldetag: 12.07.2024
(51) Int. Cl.: G16H 20/40, G16H 30/40, G16H 50/20, A61B 5/00, G06N 3/045, G06N 3/08

(54) **VERFAHREN ZUM UNTERSTÜTZEN EINER NEUROCHIRURGISCHEN OPERATION UND ANORDNUNG ZUM UNTERSTÜTZEN EINER NEUROCHIRURGISCHEN OPERATION**

(71) Anmelder: Carl Zeiss Meditec AG, 07745 Jena (DE); Medizinische Universität Wien, 1090 Wien (AT)
(72) Erfinder: Dorfer, Christian, 1090 Wien (AT); Saur, Stefan, 73447 Oberkochen (DE)
(74) Vertreter: Patentanwälte Bressel und Partner mbB

(57) **Zusammenfassung**

Die Erfindung betrifft ein Verfahren zum Unterstützen einer neurochirurgischen Operation, wobei mindestens eine Abbildung (10) eines Operationsbereichs (21) am Patienten (20) mittels eines medizinischen Visualisierungssystems (3) erfasst wird, wobei eine kategoriale Lageinformation (11), die eine anatomische Lage eines im Operationsbereich (21) vorhandenen Tumors (22) beschreibt, erfasst und/oder erhalten wird, wobei die mindestens eine erfasste Abbildung (10) und die erfasste und/oder erhaltene kategoriale Lageinformation (11) einem trainierten Maschinenlernmodell (5) als Eingangsdaten zugeführt werden, wobei ein Tumortyp (12) ausgehend von der mindestens einen erfassten Abbildung (10) und der erfassten und/oder erhaltenen kategorialen Lageinformation (11) mittels des trainierten Maschinenlernmodells (5) vorhergesagt wird, wobei das trainierte Maschinenlernmodell (5) eine den Tumortyp (12) beschreibende Tumortypinformation (13) als Ausgangsdaten bereitstellt, und wobei die Tumortypinformation (13) ausgegeben wird. Ferner betrifft die Erfindung eine Anordnung (1) zum Unterstützen einer neurochirurgischen Operation.

## Beschreibung

Die Erfindung betrifft ein Verfahren zum Unterstützen einer neurochirurgischen Operation und eine Anordnung zum Unterstützen einer neurochirurgischen Operation.

In Abhängigkeit von der Art eines Tumors muss eine geeignete mikrochirurgische Strategie (Grad der Resektion) gewählt werden. Beispielsweise werden Glioblastome sehr aggressiv entfernt, wohingegen bei Metastasen Tumorränder verbleiben können, da diese im Nachgang der Operation mittels einer Strahlentherapie oder medikamentös behandelt werden können. Lymphome werden sogar gar nicht reseziert.

Selbst mit modernen präoperativen Bildgebungsverfahren (MRT, CT etc.) können nicht alle Tumorarten differenziert werden, sodass ein Chirurg mit einer Shortlist potenzieller Tumorarten (z.B. Glioblastom und Metastase) in die Operation startet. In der Neurochirurgie wird die Tumorart schließlich erst durch eine Biopsie bestimmt, bei welcher Gewebe entnommen wird und im Anschluss an die Operation (post-operativ) neuropathologisch untersucht wird. Für ein erstes Ergebnis noch während der Operation wird heutzutage eine histopathologische Schnellschnittuntersuchung vorgenommen, die 20 bis 30 Minuten Wartezeit während des Eingriffes bedeutet. Dies führt zu einer Erhöhung der Kosten der Operation und zu einer Erhöhung des Risikos für den Patienten durch eine verlängerte Narkose. Darüber hinaus ist die Aussagekraft der histopathologischen Schnellschnittuntersuchung mit einer Genauigkeit von -90 % eingeschränkt. Alternativ oder zusätzlich zur Biopsie können auch Messsysteme im Operationssaal basierend auf z.B. der Ramanspektroskopie oder konfokaler Endomikroskopie zum Einsatz kommen. Dies erfordert jedoch zusätzliche Hardware, was einerseits Investitionskosten erzeugt und andererseits auch Platz im Operationssaal benötigt, der üblicherweise begrenzt zur Verfügung steht.

Die WO 2023/156406 A1 beschreibt ein System, das einen oder mehrere Prozessoren und ein oder mehrere Speichergeräte umfasst, zum Trainieren eines maschinellen Lernalgorithmus, wobei das System konfiguriert ist, um Trainingsdaten zu empfangen, wobei die Trainingsdaten Bilder umfassen, die Gewebe zeigen; den maschinellen Lernalgorithmus basierend auf den Trainingsdaten so anzupassen, dass der maschinelle Lernalgorithmus Anweisungsdaten für zumindest einen Teil des in den Bildern gezeigten Gewebes erzeugt, wobei die Anweisungsdaten eine an dem Gewebe auszuführende Aktion angeben; und den trainierten maschinellen Lernalgorithmus bereitzustellen. Ferner ist ein System zur Anwendung eines solchen maschinellen Lernalgorithmus und entsprechende Verfahren beschrieben.

Die US 2016 / 0 035 093 A1 beschreibt ein multimodales Brain Mapping System (MBMS), das ein oder mehrere Skope (z. B. Mikroskope oder Endoskope) umfasst, die mit einem oder mehreren Prozessoren verbunden sind, wobei die ein oder mehreren Prozessoren Trainingsdaten aus einem oder mehreren ersten Bildern und/oder ersten Daten erhalten, wobei ein oder mehrere abnormale Bereiche und ein oder mehrere normale Bereiche identifiziert werden; ferner ein zweites Bild empfangen, das von einem oder mehreren der Endoskope zu einem späteren Zeitpunkt als die ein oder mehreren ersten Bilder und/oder ersten Daten aufgenommen wurde und/oder unter Verwendung einer anderen Bildgebungstechnik aufgenommen wurde; und unter Verwendung von maschinellem Lernen, das unter Verwendung der Trainingsdaten trainiert wurde, einen oder mehrere sichtbare Indikatoren generieren, die eine oder mehrere Abnormalitäten im zweiten Bild identifizieren, wobei die ein oder mehrere sichtbaren Indikatoren in Echtzeit generiert werden, während das zweite Bild erstellt wird. Ein oder mehrere Skope zeigen einen oder mehrere sichtbare Indikatoren auf dem zweiten Bild an.

Aus Efecan Cekic et al., Deep Learning-Assisted Segmentation and Classification of Brain Tumor Types on Magnetic Resonance and Surgical Microscope Images, World Neurosurgery, E1-E9, 2023, ist eine Klassifikation von Hirntumortypen basierend auf MRT- und Mikroskopbildern bekannt.

Aus Quinn T. Ostrom et al., CBTRUS Statistical Report: Primary Brain and Other Central Nervous System Tumors Diagnosed in the United States in 2016-2020, Neuro-Oncology, Band 25, Ergänzungsausgabe 4, Oktober 2023, Seiten iv1-iv99, https://doi.org/10.1093/neuonc/noad149, sind statistische Informationen zur Häufigkeit von Tumortypen in der Neurochirurgie bekannt.

Der Erfindung liegt die Aufgabe zu Grunde, eine Lösung vorzuschlagen, bei der ein Tumortyp während des neurochirurgischen Eingriffs, insbesondere aufwandsarm und kostengünstig, auf robuste und zuverlässige Weise bestimmt werden kann.

Die Aufgabe wird erfindungsgemäß durch ein Verfahren mit den Merkmalen des Patentanspruchs 1 und eine Anordnung mit den Merkmalen des Patentanspruchs 15 gelöst. Vorteilhafte Ausgestaltungen der Erfindung ergeben sich aus den Unteransprüchen.

Es ist einer der Grundgedanken der Erfindung, einen Tumortyp ausgehend von mindestens einer erfassten Abbildung eines medizinischen Visualisierungssystems mittels eines trainierten Maschinenlernmodells vorherzusagen. Ein weiterer Grundgedanke der Erfindung ist, hierbei eine kategoriale Lageinformation zu berücksichtigen, indem die kategoriale Lageinformation erfasst und/oder erhalten wird und dem trainierten Maschinenlernmodell ebenfalls als Eingangsdaten zugeführt wird. Die kategoriale Lageinformation beschreibt eine anatomische Lage eines im Operationsbereich vorhandenen Tumors. Es hat sich gezeigt, dass die (kategoriale) anatomische Lage stark mit einem Tumortyp korreliert, sodass eine Vorhersagegenauigkeit durch Berücksichtigung der kategorialen Lageinformation deutlich verbessert werden kann. Das trainierte Maschinenlernmodell stellt eine den Tumortyp beschreibende Tumortypinformation als Ausgangsdaten bereit. Die Tumortypinformation wird ausgegeben. Es kann vorgesehen sein, dass die Tumortypinformation dem Chirurgen und/oder einer Hilfsperson auf einer Anzeigeeinrichtung angezeigt wird. Ausgehend von der ausgegebenen, insbesondere angezeigten, Tumortypinformation kann der Chirurg dann die geeignete mikrochirurgische Strategie (Grad der Resektion) wählen.

Insbesondere wird ein Verfahren zum Unterstützen einer neurochirurgischen Operation zur Verfügung gestellt, wobei mindestens eine Abbildung eines Operationsbereichs am Patienten mittels eines medizinischen Visualisierungssystems erfasst wird, wobei eine kategoriale Lageinformation, die eine anatomische Lage eines im Operationsbereich vorhandenen Tumors beschreibt, erfasst und/oder erhalten wird, wobei die mindestens eine erfasste Abbildung und die erfasste und/oder erhaltene kategoriale Lageinformation einem trainierten Maschinenlernmodell als Eingangsdaten zugeführt werden, wobei ein Tumortyp ausgehend von der mindestens einen erfassten Abbildung und der erfassten und/oder erhaltenen kategorialen Lageinformation mittels des trainierten Maschinenlernmodells vorhergesagt wird, wobei das trainierte Maschinenlernmodell eine den Tumortyp beschreibende Tumortypinformation als Ausgangsdaten bereitstellt, und wobei die Tumortypinformation ausgegeben wird.

Ferner wird insbesondere eine Anordnung zum Unterstützen einer neurochirurgischen Operation geschaffen, umfassend eine Datenverarbeitungseinrichtung, wobei die Datenverarbeitungseinrichtung dazu eingerichtet ist, mindestens eine mittels eines medizinischen Visualisierungssystems erfasste Abbildung eines Operationsbereichs am Patienten zu erhalten, eine kategoriale Lageinformation, die eine anatomische Lage eines im Operationsbereich vorhandenen Tumors beschreibt, zu erhalten, ein trainiertes Maschinenlernmodell bereitzustellen, die mindestens eine erfasste Abbildung und die erhaltene kategoriale Lageinformation dem trainierten Maschinenlernmodell als Eingangsdaten zuzuführen und einen Tumortyp ausgehend von der mindestens einen erfassten Abbildung und der erhaltenen kategorialen Lageinformation mittels des trainierten Maschinenlernmodells vorherzusagen, wobei das trainierte Maschinenlernmodell eine den Tumortyp beschreibende Tumortypinformation als Ausgangsdaten bereitgestellt, und die Tumortypinformation auszugeben.

Neben der Beseitigung der Nachteile des Stands der Technik, ist ein weiterer Vorteil des Verfahrens und der Anordnung insbesondere, dass aufgrund der Nutzung der kategorialen Lageinformation präoperative Bilddaten nicht (mit einem Maschinenlernmodell) ausgewertet werden müssen und somit ein im Hinblick auf notwendige Ressourcen (Rechenleistung und Speicher) schlankere Ausgestaltung (insbesondere des Maschinenlernmodells) ausreichend ist. Ferner sind die kategorialen Lageinformationen insbesondere auch nicht zuverlässig auf/in den präoperativen Bilddaten ersichtlich. Aus den präoperativen Bilddaten kann eine Position im (dortigen) Sichtfeld zwar gut ermittelt werden. Dies erlaubt aber nicht unbedingt eine Zuordnung zu einer anatomischen Lage, weil es unter Umständen keinen Bezug zu übergeordneten anatomischen Strukturen zulässt. Zudem ist die kategoriale Lageinformation durch den Arzt selbst einfach bestimmbar bzw. im klinischen Workflow ohnehin bereits vorhanden. Da die Auswertung prä-operativer Bilder mittels Verfahren des Maschinenlernens stets auch mit Unsicherheiten einhergeht, können durch Wegfall der Auswertung darüber hinaus Fehler vermieden werden. Die mindestens eine kategoriale Lageinformation bewirkt ferner implizit eine Normierung auf die Anatomie. So kann ein vorgegebener Voxel in einem MRT-Bild (z.B. Voxel 10110110) bei unterschiedlichen Patienten in Abhängigkeit von beispielsweise einer Lagerung des Patienten und/oder eines Erfassungsbereichs unterschiedliche anatomische Regionen zeigen.

Kategoriale Daten haben im Gegensatz zu metrischen Daten keine intervallskalierten numerischen Werte, die Rechenoperationen zulassen. Kategoriale Merkmale werden durch Nominal- und Ordinalskalen beschrieben, vorliegend insbesondere durch mindestens eine Nominalskala. Eine kategoriale Lageinformation beinhaltet insbesondere eine anatomische Lage auf einer Nominalskala. Die Nominalskala bezeichnet bzw. betrifft insbesondere (anatomische) Bereiche im menschlichen Körper. Zum Zwecke der einfacheren Verarbeitung durch das Maschinenlernmodell können die (anatomischen) Bereiche insbesondere als Zahlenwerte ausgedrückt bzw. kodiert werden, wobei die Eigenschaften der Nominalskala bestehen bleiben.

Ein medizinisches Visualisierungssystem ist insbesondere ein Operationsmikroskop. Das Operationsmikroskop kann beispielsweise ein digitales Operationsmikroskop sein, insbesondere mit einer digitalen Visualisierungskette, bei der eine Abbildung auf (zumindest) einen Kamerasensor stattfindet. Weiter kann das Operationsmikroskop ein analoges Operationsmikroskop sein, welches insbesondere einen_optischen Pfad mit Linsen und Okular(en) aufweist. Ferner kann das Operationsmikroskop ein hybrides Operationsmikroskop sein. Grundsätzlich kann ein medizinisches Visualisierungssystem aber auch ein anderes medizinisches System sein, welches die mindestens eine Abbildung vom Operationsbereich erfassen kann, wie beispielsweise ein Mikro-Inspektionstool oder ein Endoskop.

Die mindestens eine Abbildung kann vom medizinischen Visualisierungssystem erfasst und/oder bereitgestellt werden. Die mindestens eine erfasste Abbildung kann eine Weißlichtabbildung und/oder eine Fluoreszenzabbildung, beispielsweise BLAU-400 oder YELLOW-560, umfassen. Es kann beispielsweise vorgesehen sein, dass eine Weißlichtabbildung und eine Fluoreszenzabbildung miteinander fusioniert werden und als (fusionierte) erfasste Abbildung verwendet werden. Zweckmäßigerweise wird die erfasste Abbildung dem trainierten Maschinenlernmodell zugeführt.

Das Maschinenlernmodell kann beispielsweise ein künstliches Neuronales Netz sein oder mindestens ein solches umfassen. Dem Maschinenlernmodell werden als Eingangsdaten die erfasste mindestens eine Abbildung und die kategoriale Lageinformation zugeführt. Als Ausgangsdaten stellt das Maschinenlernmodell eine Vorhersage des Tumortyps bereit. Das Maschinenlernmodell ist bzw. wurde insbesondere mit Hilfe von Trainingsdaten trainiert. Hierbei umfassen die Trainingsdaten insbesondere eine Vielzahl von Paaren, die jeweils eine den Tumor zeigende Abbildung und eine die anatomische Lage des abgebildeten Tumors beschreibende kategoriale Lageinformation, gepaart mit einer Tumortypinformation als Annotation bzw. als Grundwahrheit (engl. ground truth), umfassen. Die Grundwahrheit kann beispielsweise mittels einer neuropathologischen Gewebeuntersuchung bestimmt werden. Das Maschinenlernmodell wird mit Hilfe der Trainingsdaten in an sich bekannter Weise trainiert, insbesondere im Wege des überwachten Lernens.

Es kann vorgesehen sein, dass das Maschinenlernmodell mit Hilfe von statistischen Metadaten zur Korrelation zwischen anatomischer Lage (in Form der kategorialen Lageinformation) und Tumortyp vortrainiert wird. Hierdurch kann das Maschinenlernmodell die Prävalenzstatistiken der Tumortypen in Abhängigkeit der anatomischen Lage erlernen. Anschließend kann in einem Feintraining (Finetuning) das Trainieren mittels der Trainingsdaten, umfassend die Abbildungen, die kategoriale Lageinformation und den jeweils als Grundwahrheit zugeordneten Tumortyp, erfolgen. Als Datenquelle für die Prävalenzen können beispielsweise statistische Berichte wie der eingangs aufgeführte Bericht (Ostrom et al.) verwendet werden.

Die Tumortypinformation kann beispielsweise Wahrscheinlichkeitswerte für mögliche Tumortypen umfassen, die von dem trainierten Maschinenlernmodell für jeden Tumortyp geschätzt wurden. Es kann dann anhand der Wahrscheinlichkeitswerte der am wahrscheinlichsten vorliegende Tumortyp bestimmt werden. Es kann jedoch auch vorgesehen sein, dass die Tumortypinformation bereits eine Aussage darüber enthält, welcher Tumortyp vorliegt, ohne dass Wahrscheinlichkeitswerte in der Tumortypinformation enthalten sind (es wird dann nur der wahrscheinlichste Tumortyp angegeben).

Es kann vorgesehen sein, dass das Verfahren erst gestartet wird, wenn ein Nutzer (Chirurg oder Hilfspersonal) den Befehl hierzu gibt. Dies kann beispielsweise erfolgen, wenn über dem Tumor liegendes Gewebe entfernt oder geöffnet wurde und der Tumor erreicht ist bzw. wenn der Tumor optisch erfasst werden kann.

Teile der Anordnung, insbesondere die Datenverarbeitungseinrichtung, können einzeln oder zusammengefasst als eine Kombination von Hardware und Software ausgebildet sein, beispielsweise als Programmcode, der auf einem Mikrocontroller oder Mikroprozessor ausgeführt wird. Es kann jedoch auch vorgesehen sein, dass Teile einzeln oder zusammengefasst als anwendungsspezifische integrierte Schaltung (ASIC) und/oder feldprogrammierbares Gatterfeld (FPGA) und/oder Graphikprozessor (GPU) und/oder Digitaler Signalprozessor (DSP) ausgebildet sind. Die Datenverarbeitungseinrichtung umfasst insbesondere mindestens eine Recheneinrichtung und mindestens einen Speicher.

In einer Ausführungsform ist vorgesehen, dass die kategoriale Lageinformation textbasiert erfasst und/oder erhalten wird. Hierdurch kann die anatomische Lageinformation auf besonders einfach für einen Menschen erfassbare Weise erfasst und/oder erhalten werden. Beispielsweise kann vorgesehen sein, dass die kategoriale Lageinformation textbasiert an einer Anzeige- und Bedieneinrichtung (GUI) erfasst wird, wobei ein Nutzer (insbesondere klinisches Personal, beispielsweise ein Chirurg oder Hilfspersonal) auf einer Tastatur die kategoriale Lageinformation eingeben kann. Ferner kann die kategoriale Lageinformation aus einer Auswahlliste, in welcher beispielsweise mehrere Kategorien für die kategoriale Lageinformation textbasiert vorgegeben sind, ausgewählt werden. Weiter kann die kategoriale Lageinformation als transkribierte Spracheingabe erfasst werden.

In einer Ausführungsform ist vorgesehen, dass zum Erfassen der kategorialen Lageinformation eine anatomische Karte angezeigt wird, wobei die kategoriale Lageinformation durch Auswahl an der anatomischen Karte erfasst wird. Hierdurch kann eine besonders anschauliche Art des Erfassens bereitgestellt werden, wodurch insbesondere eine Erfassungsgeschwindigkeit erhöht werden kann und Fehleingaben verhindert oder zumindest verringert werden können. Beispielsweise kann vorgesehen sein, die anatomische Karte auf einer Anzeigeeinrichtung oder einer Anzeige- und Bedieneinrichtung anzuzeigen. Ein Nutzer (Chirurg oder Hilfspersonal) kann die kategoriale Lageinformation durch Auswählen der anatomischen Lage auf der anatomischen Karte festlegen.

In einer Ausführungsform ist vorgesehen, dass die kategoriale Lageinformation eine Information zum Hirnlappen umfasst, der den Tumor beinhaltet. Hierdurch kann die Güte bei der Vorhersage gesteigert werden. Insbesondere umfasst die Information zum Hirnlappen eine der folgenden Angaben: Frontallappen (lobus frontalis), Temporallappen/Schläfenlappen (lobus temporalis), Parietallappen/Scheitellappen (lobus parietalis), Okzipitallappen/Hinterhauptslappen (lobus occipitalis).

In einer Ausführungsform ist vorgesehen, dass die kategoriale Lageinformation eine Information zur Gehirnhälfte umfasst, die den Tumor beinhaltet. Hierdurch kann die Güte bei der Vorhersage gesteigert werden. Insbesondere umfasst die Information zur Gehirnhälfte eine der folgenden Angaben: linke Hemisphäre, rechte Hemisphäre.

In einer Ausführungsform ist vorgesehen, dass die kategoriale Lageinformation eine Information zur Hirnregion umfasst, die den Tumor beinhaltet. Hierdurch kann die Güte bei der Vorhersage gesteigert werden. Insbesondere umfasst die Information zur Hirnregion eine der folgenden Angaben: supratentoriell, supratentoriell Mittellinie, infratentoriell, periventrikulär, spinal (also im Grenzbereich zwischen Gehirn und Wirbelsäule), zentral.

Ferner kann vorgesehen sein, dass die Information zusätzlich oder alternativ eine der folgenden Informationen für eine Position innerhalb der Hirnlappen, Gehirnhälfte und/oder Hirnregion umfasst: anterior vs. posterior; medial vs. lateral, kortikal/subkortikal (oberflächlich) oder tief; Mittellinie oder nicht.

In einer Ausführungsform ist vorgesehen, dass die kategoriale Lageinformation mindestens eine relationale Information umfasst, die den Tumor in Bezug zu anatomischen Merkmalen verortet. Hierdurch kann eine kategoriale Angabe noch genauer erfolgen. Eine Güte der Vorhersage kann hierdurch weiter gesteigert werden. Beispielsweise kann vorgesehen sein, dass die relationale Information Angaben in Bezug auf eine Lage zu einem Gefäßsystem des Gehirns beinhaltet, beispielsweise eine Angabe, dass der Tumor zwischen zwei Gefäßen (mit entsprechender anatomischer Bezeichnung) liegt oder dass der Tumor ein Gefäß (mit anatomischer Angabe) berührt etc. Es kann vorgesehen sein, dass die relationale Information mit Bezug auf einen digitalen Zwilling (zumindest) eines Teils des Patienten oder eines generischen digitalen Zwillings (zumindest) eines Teils des menschlichen Körpers angegeben wird.

In einer Ausführungsform ist vorgesehen, dass zusätzlich mindestens ein visueller Indikator erzeugt und als Bildsignal ausgegeben wird, wobei der visuelle Indikator zumindest einen Bereich in der erfassten mindestens einen Abbildung kennzeichnet, der beim Vorhersagen berücksichtigt wurde. Der visuelle Indikator kann beispielsweise ausgehend von Daten des trainierten Maschinenlernmodells erzeugt bzw. bestimmt werden. Beispielsweise können hierzu sogenannte Aufmerksamkeitskarten (engl. attention maps, vgl. z.B. Jungkan An et al., Attention Map-Guided Visual Explanations for Deep Neural Networks, Appl. Sci. 2022, 12(8), 3846;
https://doi.org/10.3390/app12083846) und/oder Klassenaktivierungskarten (engl. class activation maps, vgl. Anh Pham Thi Minh, Overview of Class Activation Maps for Visualization Explainability, arXiv:2309.14304 [cs.CV], 2023,
https://doi.org/10.48550/arXiv.2309.14304) verwendet werden. Es kann vorgesehen sein, dass der visuelle Indikator zusammen mit der mindestens einen Abbildung angezeigt wird, beispielsweise indem der visuelle Indikator über dem jeweiligen Bereich der mindestens eine Abbildung dargestellt wird.

In einer Ausführungsform ist vorgesehen, dass mindestens eine Patienteninformation erfasst und/oder erhalten wird, wobei dem trainierten Maschinenlernmodell zusätzlich die mindestens eine erfasste und/oder erhaltene Patienteninformation als Eingangsdaten zugeführt wird, und wobei das trainierte Maschinenlernmodell den Tumortyp zusätzlich unter Berücksichtigung der mindestens einen Patienteninformation vorhersagt. Hierdurch kann eine Güte der Vorhersage weiter verbessert werden, da das Auftreten von Tumortypen insbesondere auch mit Eigenschaften der Patienten korreliert. Beispielsweise kann eine Patienteninformation ein der folgenden betreffen: Alter, Geschlecht, eine Angabe darüber, ob sich ein Kontrastmittel angelagert hat und/oder eine Historie der Erkrankung (z.B. Auftreten einer Epilepsie oder ob bereits ein anderer primärer Tumor, beispielsweise an der gleichen Stelle, im Patienten diagnostiziert wurde etc.). Beim Trainieren des Maschinenlernmodells umfassen die hierbei verwendeten Trainingsdaten insbesondere auch die entsprechende mindestens eine Patienteninformation.

Es kann vorgesehen sein, dass das Maschinenlernmodell mittels statistischer Metadaten, welche die mindestens eine Patienteninformation, gepaart mit den jeweiligen Häufigkeiten der Tumortypen, umfassen, vortrainiert wird, wie dies voranstehend bereits beschrieben wurde.

Es kann vorgesehen sein, dass zusätzlich präoperative Daten des Operationsbereichs (z.B. MRT, CT etc.) berücksichtigt werden. Die präoperativen Daten können beispielsweise als Patienteninformation berücksichtigt werden. Die präoperativen Daten werden dem trainierten Maschinenlernmodell ebenfalls als Eingangsdaten zugeführt und bei der Vorhersage des Tumortyps berücksichtigt. Die Trainingsdaten umfassen dann entsprechende präoperative Daten.

In einer Ausführungsform ist vorgesehen, dass das trainierte Maschinenlernmodell ein Faltungsnetz, ein mehrschichtiges Perceptron und ein Klassifizierungsnetz aufweist, wobei die erfasste und/oder erhaltene mindestens eine Abbildung mittels des Faltungsnetzes in ein ersten Embeddingteil überführt wird, wobei die kategoriale Lageinformation und/oder die mindestens eine Patienteninformation mittels des mehrschichtigen Perceptrons in einen zweiten Embeddingteil überführt wird, und wobei der erste Embeddingteil und der zweite Embeddingteil dem Klassifikationsnetz als Eingangsdaten zugeführt werden. Hierdurch kann eine besonders hohe Güte bei der Vorhersage erzielt werden.

Grundsätzlich kann das Maschinenlernmodell jedoch auch eine andere Architektur aufweisen, beispielsweise eine Transformer-Architektur. Ferner können auch Ansätze aus dem Bereich der Multi-Sensor-Fusion verwendet werden (vgl. Benedict Marsh et al, A Critical Review of Deep Learning-Based Multi-Sensor Fusion Techniques, Sensors 2022, 22(23), 9364; https://doi.org/10.3390/s22239364).

In einer Ausführungsform ist vorgesehen, dass Geräuschdaten eines während der neurochirurgischen Operation verwendeten Saugers erfasst und/oder erhalten werden, wobei dem trainierten Maschinenlernmodell zusätzlich die erfassten und/oder erhaltenen Geräuschdaten als Eingangsdaten zugeführt werden, und wobei das trainierte Maschinenlernmodell den Tumortyp zusätzlich unter Berücksichtigung der erfassten und/oder erhaltenen Geräuschdaten vorhersagt. Hierdurch kann die Güte bei der Vorhersage weiter erhöht werden, da aufgrund einer unterschiedlichen Gewebeart auch die Geräuschinformationen mit dem Tumortyp korreliert. Die Geräuschdaten umfassen insbesondere aufgezeichnete Geräuschsignale, die beispielsweise mittels eines Mikrofons erfasst werden. Beim Trainieren des Maschinenlernmodells umfassen die hierbei verwendeten Trainingsdaten insbesondere auch entsprechende Geräuschdaten.

In einer Ausführungsform ist vorgesehen, dass eine Elastizität eines Gewebes abbildende haptische Messdaten im Operationsbereich erfasst und/oder erhalten werden, wobei dem trainierten Maschinenlernmodell zusätzlich die erfassten und/oder erhaltenen haptischen Messdaten als Eingangsdaten zugeführt werden, und wobei das trainierte Maschinenlernmodell den Tumortyp zusätzlich unter Berücksichtigung der erfassten und/oder erhaltenen haptischen Messdaten vorhersagt. Hierdurch kann eine Güte beim Vorhersagen des Tumortyps weiter gesteigert werden. Beim Trainieren des Maschinenlernmodells umfassen die hierbei verwendeten Trainingsdaten insbesondere auch entsprechende haptische Messdaten. Die haptischen Messdaten können beispielsweise mittels piezoelektrischer Sensoren erfasst werden (vgl. z.B. Jörg Wallaschek et al., Mechatronischer Tastsinn - Wie piezoelektrische taktile Sensoren Diagnosen optimieren, AlumniCampus 4, 2010, https://www.uni-hannover.de/fileadmin/luh/content/alumni/alumnicampus/AC_4_2010/48-51_forsch10_wallaschek.pdf).

In einer Ausführungsform ist vorgesehen, dass eine Liste von Tumortypen erfasst und/oder vorgegeben wird, wobei dem trainierten Maschinenlernmodell zusätzlich die Liste von Tumortypen als Eingangsdaten zugeführt wird, und wobei das trainierte Maschinenlernmodell den Tumortyp zusätzlich unter Berücksichtigung der Liste von Tumortypen vorhersagt. Hierdurch kann eine Güte beim Vorhersagen gesteigert werden, weil das trainierte Maschinenlernmodell zusätzliche Informationen zu den möglicherweise vorliegenden Tumortypen erhält, eine Entscheidung also nur zwischen einer begrenzten Anzahl von Tumortypen treffen muss bzw. nur für die begrenzte Anzahl Wahrscheinlichkeiten schätzen muss. Beim Trainieren des Maschinenlernmodells umfassen die hierbei verwendeten Trainingsdaten insbesondere auch entsprechende Listen.

In einer Ausführungsform ist vorgesehen, dass eine Liste von Tumortypen erfasst und/oder vorgegeben wird, wobei für verschiedene Listen von Tumortypen zumindest gruppenweise voneinander verschiedene trainierte Maschinenlernmodelle verwendet werden, wobei das jeweils passende trainierte Maschinenlernmodell ausgehend von der erfassten und/oder vorgegebenen Liste von Tumortypen ausgewählt wird. Hierdurch kann eine Güte bei der Vorhersage weiter gesteigert werden, da für verschiedene Listen jeweils ein spezialisiertes Maschinenlernmodell bereitgestellt werden kann. Das jeweils passende bzw. spezialisierte Maschinenlernmodell schätzt dann den Tumortyp insbesondere ausschließlich für die in der zugehörigen Liste enthaltenen Tumortypen. Es kann jedoch auch vorgesehen sein, dass für Listen, die in Bezug auf Tumortypen Schnittmengen miteinander aufweisen, gemeinsam genutzte Maschinenlernmodelle verwendet werden, das heißt, grundsätzlich können mehrere Listen sich ein gemeinsames Maschinenlernmodell teilen.

In einer weiterbildenden Ausführungsform ist vorgesehen, dass die Liste zwei Tumortypen umfasst. Hierdurch kann der höchste Grad an Spezialisierung der trainierten Maschinenlernmodelle erfolgen, sodass auch eine Güte der Vorhersage weiter erhöht werden kann. Insbesondere kann vorgesehen sein, dass für jede Kombination von zwei Tumortypen jeweils ein trainiertes Maschinenlernmodell bereitgestellt und verwendet wird.

In einer Ausführungsform ist vorgesehen, dass ausgehend von dem vorhergesagten Tumortyp mindestens ein Steuersignal erzeugt und/oder bereitgestellt wird, das dazu eingerichtet ist, das medizinische Visualisierungssystem und/oder ein anderes medizinisches Gerät anzusteuern. Hierdurch kann ein Chirurg bei der anstehenden Phase der Operation (insbesondere der Tumorresektion) weitergehend unterstützt werden. Beispielsweise kann das mindestens eine Steuersignal dazu dienen, Einstellungen des medizinischen Visualisierungssystems zu ändern und/oder ein anderes medizinisches Gerät zu aktivieren und/oder vorzubereiten.

Es kann vorgesehen sein, dass das Maschinenlernmodell mit Hilfe von Biopsiedaten, die den jeweiligen (labortechnisch) bestimmten Tumortyp enthalten, nachtrainiert wird. Die Biopsiedaten dienen dann als Grundwahrheit für die jeweils zugehörig erfasste Abbildung.

Weitere Merkmale zur Ausgestaltung der Anordnung ergeben sich aus der Beschreibung von Ausgestaltungen des Verfahrens. Die Vorteile der Anordnung sind hierbei jeweils die gleichen wie bei den Ausgestaltungen des Verfahrens.

Nachfolgend wird die Erfindung anhand bevorzugter Ausführungsbeispiele unter Bezugnahme auf die Figuren näher erläutert. Hierbei zeigen:
- Fig. 1: eine schematische Darstellung einer Ausführungsform der Anordnung zum Unterstützen einer neurochirurgischen Operation;
- Fig. 2: eine beispielhafte Ausführungsform des Maschinenlernmodells;
- Fig. 3: ein schematisches Ablaufdiagramm einer Ausführungsform des Verfahrens.

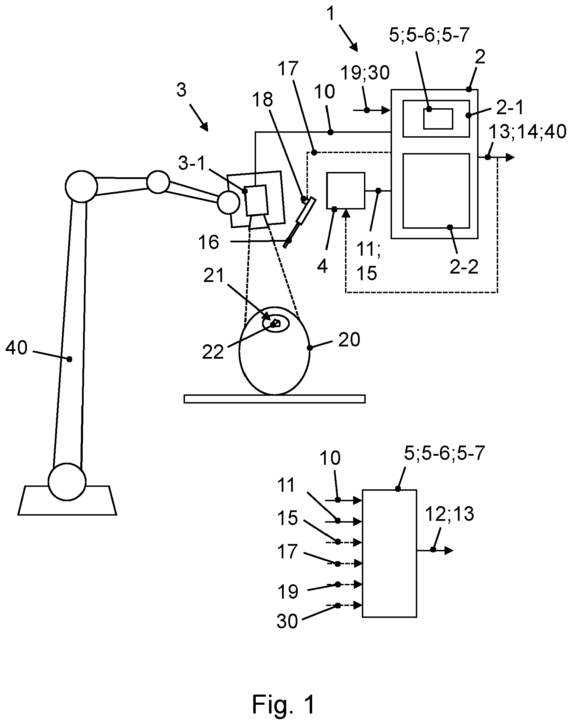

Die Fig. 1 zeigt eine schematische Darstellung einer Ausführungsform der Anordnung 1 zum Unterstützen einer neurochirurgischen Operation. Die Anordnung 1 ist dazu eingerichtet, das in dieser Offenbarung beschriebene Verfahren auszuführen. Das Verfahren wird nachfolgend anhand der Anordnung 1 näher beschrieben.

Die Anordnung 1 umfasst eine Datenverarbeitungseinrichtung 2. Die Datenverarbeitungseinrichtung 2 umfasst mindestens eine Recheneinrichtung 2-1 und mindestens einen Speicher 2-2. Die Recheneinrichtung 2-1 ist dazu eingerichtet zum Durchführen des Verfahrens notwendige Rechenoperationen auszuführen, und kann hierzu auf im Speicher 2-2 hinterlegte Daten zugreifen.

Die Datenverarbeitungseinrichtung 2 ist dazu eingerichtet, mindestens eine mittels eines medizinischen Visualisierungssystems 3, beispielsweise einem Operationsmikroskop, insbesondere mittels einer Kamera 3-1, erfasste Abbildung 10 eines Operationsbereichs 21 am Patienten 20 zu erhalten. Das medizinische Visualisierungssystem 3 kann auch Teil der Anordnung 1 sein. Ferner kann das medizinische Visualisierungssystem 3 die Anordnung 1 auch umfassen. Weiter erhält die Datenverarbeitungseinrichtung 2 eine kategoriale Lageinformation 11, die eine anatomische Lage eines im Operationsbereich 21 vorhandenen Tumors 22 beschreibt. Die kategoriale Lageinformation 11 kann beispielsweise mittels einer Anzeige- und Bedieneinrichtung 4 erfasst werden. Die Anzeige- und Bedieneinrichtung 4 kann Teil der Anordnung 1 sein. Alternativ kann die kategoriale Lageinformation 11 auch von einem Krankenhausinformationssystem (KIS) bereitgestellt werden und/oder aus diesem mittels der Datenverarbeitungseinrichtung 2 abgerufen werden. Insbesondere kann die kategoriale Lageinformation 11 aus einer elektronischen Patientenakte (engl. electronic health record, EHR) bereitgestellt und/oder abgerufen werden.

Die Datenverarbeitungseinrichtung 2 ist dazu eingerichtet, ein trainiertes Maschinenlernmodell 5 bereitzustellen, die mindestens eine erfasste Abbildung 10 und die erhaltene kategoriale Lageinformation 11 dem trainierten Maschinenlernmodell 5 als Eingangsdaten zuzuführen und einen Tumortyp 12 ausgehend von der mindestens einen erfassten Abbildung 10 und der erhaltenen kategorialen Lageinformation 11 mittels des trainierten Maschinenlernmodells 5 vorherzusagen. Das trainierte Maschinenlernmodell 5 stellt an einem Ausgang eine den Tumortyp 12 beschreibende Tumortypinformation 13 als Ausgangsdaten bereit. Die Datenverarbeitungseinrichtung 2 gibt die Tumortypinformation 13 aus. Beispielsweise kann die Datenverarbeitungseinrichtung 2 die Tumortypinformation 13 an eine Anzeigeeinheit, z.B. an die Anzeige- und Bedieneinrichtung 4 oder ein (sonstiges) Display, ausgeben. Die Anzeigeeinheit kann eine Anzeigeeinheit eines Operationsmikroskops oder ein externer 2D- oder 3D-Monitor sein. Ferner kann auch eine Überlagerung in ein digitales Okular oder in einer AR- oder VR-Brille (engl. Head-Mounted-Display, HMD) erfolgen.

Es ist insbesondere vorgesehen, dass die Tumortypinformation 13 angezeigt wird, beispielsweise auf der Anzeige- und Bedieneinrichtung 4. Die Tumortypinformation 13 kann beispielsweise als den Tumortyp umfassende Textinformation angezeigt werden. Es kann vorgesehen sein, dass mit dem Vorliegen verschiedener Tumortypen 12 einhergehende Wahrscheinlichkeiten ebenfalls ausgegeben und/oder angezeigt werden. Ein Chirurg kann dann ausgehend von der angezeigten Tumortypinformation entscheiden, wie die Operation fortgeführt werden soll, insbesondere welcher Grad der Resektion für den vorliegenden Tumor 22 gewählt wird.

Es kann vorgesehen sein, dass die kategoriale Lageinformation 11 textbasiert erfasst und/oder erhalten wird. Beispielsweise kann vorgesehen sein, die kategoriale Lageinformation 11 direkt als Text mittels einer Tastatur zu erfassen, beispielsweise mittels der Anzeige- und Bedieneinrichtung 4. Alternativ oder zusätzlich kann auch vorgesehen sein, dass die kategoriale Lageinformation 11 textbasiert aus einer Liste ausgewählt werden kann, beispielsweise mittels der Anzeige- und Bedieneinrichtung 4.

Es kann alternativ oder zusätzlich vorgesehen sein, dass zum Erfassen der kategorialen Lageinformation 11 eine anatomische Karte angezeigt wird, wobei die kategoriale Lageinformation 11 durch Auswahl an der anatomischen Karte erfasst wird. Es kann beispielsweise vorgesehen sein, dass die anatomische Karte auf der Anzeige- und Bedieneinrichtung 4 angezeigt wird, wobei anatomische Bereiche auswählbar sind, beispielsweise indem diese durch Anklicken und/oder Berührung eines hiermit korrespondierenden Bereichs auf der Anzeige- und Bedieneinrichtung 4 ausgewählt werden.

Es kann vorgesehen sein, dass die kategoriale Lageinformation 11 eine Information zum Hirnlappen umfasst, der den Tumor beinhaltet.

Es kann vorgesehen sein, dass die kategoriale Lageinformation 11 eine Information zur Gehirnhälfte umfasst, die den Tumor beinhaltet.

Es kann vorgesehen sein, dass die kategoriale Lageinformation 11 eine Information zur Hirnregion umfasst, die den Tumor beinhaltet.

Es kann vorgesehen sein, dass die kategoriale Lageinformation 11 mindestens eine relationale Information umfasst, die den Tumor in Bezug zu anatomischen Merkmalen verortet.

Es kann vorgesehen sein, dass zusätzlich mindestens ein visueller Indikator 14 erzeugt und als Bildsignal ausgegeben wird, wobei der visuelle Indikator 14 zumindest einen Bereich in der erfassten mindestens einen Abbildung 10 kennzeichnet, der beim Vorhersagen berücksichtigt wurde. Es kann beispielsweise vorgesehen sein, dass der mindestens eine visuelle Indikator 14 auf der Anzeige- und Bedieneinrichtung 4 angezeigt wird, insbesondere zusammen mit der erfassten mindestens einen Abbildung 10.

Es kann vorgesehen sein, dass mindestens eine Patienteninformation 15 erfasst und/oder erhalten wird, wobei dem trainierten Maschinenlernmodell 5 zusätzlich die mindestens eine erfasste und/oder erhaltene Patienteninformation 15 als Eingangsdaten zugeführt wird, wobei das trainierte Maschinenlernmodell 15 den Tumortyp 12 zusätzlich unter Berücksichtigung der mindestens einen Patienteninformation 15 vorhersagt. Die mindestens einen Patienteninformation 15 kann beispielsweise aus einer digitalen Patientenakte extrahiert werden. Beispielsweise kann vorgesehen sein, die mindestens eine Patienteninformation 15 aus einer Datenbank abzufragen.

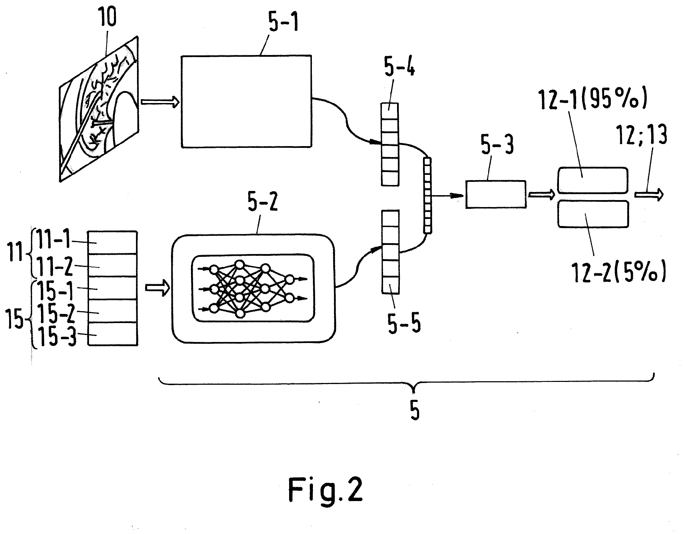

Die Fig. 2 zeigt eine beispielhafte Ausführungsform des Maschinenlernmodells 5. Es ist bei dieser Ausführungsform vorgesehen, dass das trainierte Maschinenlernmodell 5 ein Faltungsnetz 5-1, ein mehrschichtiges Perceptron 5-2 und ein Klassifizierungsnetz 5-3 aufweist, wobei die erfasste und/oder erhaltene mindestens eine Abbildung 10 mittels des Faltungsnetzes 5-1 in ein ersten Embeddingteil 5-4 überführt wird, wobei die kategoriale Lageinformation 11 und/oder die mindestens eine Patienteninformation 15 mittels des mehrschichtigen Perceptrons 5-2 in einen zweiten Embeddingteil 5-5 überführt wird, und wobei der erste Embeddingteil 5-4 und der zweite Embeddingteil 5-5 dem Klassifikationsnetz 5-3 als Eingangsdaten zugeführt werden. Die kategoriale Lageinformation 11 umfasst beispielsweise eine Angabe zum Hirnlappen 11-1 und eine Angabe zur Hirnregion 11-2 (Seite). Die mindestens eine Patienteninformation 15 umfasst beispielsweise ein Geschlecht 15-1, ein Alter 15-2 und eine Historie 15-3 zum Tumor. Es kann vorgesehen sein, dass das mehrschichtige Perceptron 5-2 vortrainiert wird mit Hilfe von statistischen Daten zur Prävalenz bzw. Häufigkeit von Tumortypen bei den jeweiligen Ausprägungen der kategorialen Lageinformation 11 und/oder der Patienteninformationen 15 (siehe weiter unten). Das Klassifikationsnetz 5-3 schätzt beispielsweise eine Wahrscheinlichkeit für das Vorliegen eines jeweiligen Tumortyps 12. Im gezeigten Beispiel soll eine Vorhersage darüber getroffen werden, ob ein Glioblastom 12-1 oder eine Metastase 12-2 vorliegt. Beispielhaft soll die Vorhersage des Klassifikationsnetzes 5-3 ergeben, dass mit einer Wahrscheinlichkeit von 95 % ein Glioblastom 12-1 vorliegt und mit einer Wahrscheinlichkeit von 5 % eine Metastase. Diese Werte können als Tumortypinformation 13 ausgegeben werden.

Zum Vortrainieren des mehrschichtigen Perceptrons 5-2 gibt es mehrere Möglichkeiten, von denen beispielhaft eine ausgeführt wird: Es können beispielsweise mittels Monte-Carlo-Sampling Datensätze erzeugt werden, die der Prävalenzstatistik folgen. Hiermit kann dann der untere Zweig trainiert werden. Hierzu wird nach dem zweiten Embeddingteil 5-5 direkt ein Klassifikator verwendet. Das Ergebnis ist eine Parametrisierung des mehrschichtigen Perceptrons 5-2. Für das Training mit echten Patientendaten (also der mindestens einen Abbildung 10 und der kategorialen Lageinformation 11) werden dann entweder die Gewichte des mehrschichtigen Perceptrons 5-2 eingefroren (alle oder nur ein Teil) und/oder eine Änderung der Gewichte wird begrenzt.

Es kann vorgesehen sein, dass Geräuschdaten 17 (Fig. 1) eines während der neurochirurgischen Operation verwendeten Saugers 16 erfasst und/oder erhalten werden, wobei dem trainierten Maschinenlernmodell 5 zusätzlich die erfassten und/oder erhaltenen Geräuschdaten 17 als Eingangsdaten zugeführt werden, und wobei das trainierte Maschinenlernmodell 5 den Tumortyp 12 zusätzlich unter Berücksichtigung der erfassten und/oder erhaltenen Geräuschdaten 17 vorhersagt. Die Geräuschdaten 17 können beispielsweise mittels eines in der Nähe oder am Sauger 16 angeordneten Mikrofons 18 erfasst werden.

Es kann vorgesehen sein, dass eine Elastizität eines Gewebes abbildende haptische Messdaten 19 im Operationsbereich 21 erfasst und/oder erhalten werden, wobei dem trainierten Maschinenlernmodell 5 zusätzlich die erfassten und/oder erhaltenen haptischen Messdaten 19 als Eingangsdaten zugeführt werden, wobei das trainierte Maschinenlernmodell 5 den Tumortyp 12 zusätzlich unter Berücksichtigung der erfassten und/oder erhaltenen haptischen Messdaten 12 vorhersagt.

Es kann vorgesehen sein, dass eine Liste 30 von Tumortypen 12 erfasst und/oder vorgegeben wird, wobei dem trainierten Maschinenlernmodell 5 zusätzlich die Liste 30 von Tumortypen 12 als Eingangsdaten zugeführt wird, und wobei das trainierte Maschinenlernmodell 5 den Tumortyp 12 zusätzlich unter Berücksichtigung der Liste 30 von Tumortypen 12 vorhersagt. Die Liste 30 von Tumortypen 12 umfasst insbesondere mehrere mögliche Tumortypen 12. Die Liste 30 kann beispielsweise als Spaltenvektor dem Maschinenlernmodell 5 übergeben werden, in dem jeder Eintrag mit einem möglichen Tumortyp 12 korrespondiert und die Auswahl der Einträge der Liste 30 über den Eintrag einer "0" (= "dieser Tumortyp ist nicht möglich") oder einer "1" (= "dieser Tumortyp ist möglich") erfolgt. Beispielsweise gibt der Spaltenvektor (0,1,0,0,1) für die Tumortypen 1 bis 5 vor, dass nur der Tumortyp 2 und der Tumortyp 5 vorhanden sein können, das trainierte Maschinenlernmodell 5 daher auch nur eine Vorhersage zur Unterscheidung dieser beiden Tumortypen treffen muss. Das Maschinenlernmodell 5 wird während einer Trainingsphase insbesondere unter Berücksichtigung entsprechender Listen 30 trainiert.

Es kann vorgesehen sein, dass eine Liste 30 von Tumortypen 12 erfasst und/oder vorgegeben wird, wobei für verschiedene Listen 30 von Tumortypen 12 zumindest gruppenweise voneinander verschiedene trainierte Maschinenlernmodelle 5 verwendet werden, wobei das jeweils passende trainierte Maschinenlernmodell 5 ausgehend von der erfassten und/oder vorgegebenen Liste 30 von Tumortypen 12 ausgewählt wird. Gibt es in einem stark vereinfachten Beispiel die Tumortypen 1 bis 4, welche jedoch nur in den Kombinationen Tumortyp 1 mit Tumortyp 2 und Tumortyp 3 mit Tumortyp 4 auftreten, so werden zwei spezialisierte Maschinenlernmodelle 5 verwendet, wobei ein erstes Maschinenlernmodell 5-6 auf die Vorhersage zur ersten Kombination und ein zweites Maschinenlernmodell 5-7 auf die Vorhersage zur zweiten Kombination spezialisiert ist. Entsprechend wird durch die Liste 30 vorgegeben, welche der Kombinationen vorliegt, z.B. in Form eines Spaltenvektors als (1,1,0,0) für die erste Kombination oder (0,0,1,1) für die zweite Kombination. Bei der ersten Kombination bzw. beim ersten Spaltenvektor wird das trainierte erste Maschinenlernmodell 5-6 verwendet, um den Tumortyp 12 vorherzusagen, bei der zweiten Kombination bzw. beim zweiten Spaltenvektor wird das trainierte zweite Maschinenlernmodell 5-7 verwendet, um den Tumortyp 12 vorherzusagen.

Es ist insbesondere vorgesehen, dass die Liste 30 zwei Tumortypen 12 umfasst. Hierdurch kann eine maximale Beschränkung der möglichen Tumortypen vorgenommen werden. Insbesondere bei Verwendung von mehreren spezialisierten Maschinenlernmodellen 5 können diese maximal auf die jeweils vorliegende Kombination spezialisiert sein. Beispiele hierfür sind: Glioblastom vs. Metastasen, Glioblastom vs. Lymphome, Lymphome vs. Astrocytome und Lymphome vs. anderes Gewebe (die Vorhersage beinhaltet also nur eine Aussage darüber, ob ein Lymphom vorliegt oder nicht).

Die Liste 30 kann beispielsweise an der Anzeige- und Bedieneinrichtung 4 oder mittels einer anderen Erfassungseinrichtung (nicht gezeigt) erfasst werden. Es kann vorgesehen sein, dass eine Auswahl von Listen 30 auf der Anzeige- und Bedieneinrichtung 4 angezeigt wird und ausgewählt werden kann. Es kann jedoch auch vorgesehen sein, dass ein Nutzer die Liste 30 durch Auswahl von möglichen Tumortypen 12 vorgeben kann.

Es kann vorgesehen sein, dass ausgehend von dem vorhergesagten Tumortyp 12 mindestens ein Steuersignal 40 erzeugt und/oder bereitgestellt wird, das dazu eingerichtet ist, das medizinische Visualisierungssystem 3 und/oder ein anderes medizinisches Gerät (nicht gezeigt) anzusteuern. Hierzu kann die Datenverarbeitungseinrichtung 2 dazu eingerichtet sein, aus dem vorhergesagten Tumortyp 12 und/oder aus der Tumortypinformation 13 mindestens ein Steuersignal 40 zu erzeugen und bereitzustellen. Das Steuersignal 40 kann analoger und/oder digitaler Art sein. Beispielsweise kann vorgesehen sein, mit Hilfe des Steuersignals 40 einen Betriebsmodus des medizinischen Visualisierungssystems 3 und/oder des anderen medizinischen Geräts einzustellen und/oder zu ändern und/oder Einstellungen zu ändern.

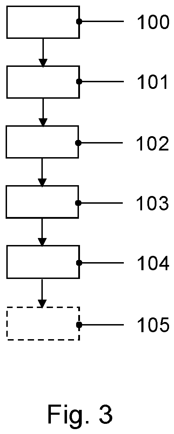

Die Fig. 3 zeigt ein schematisches Ablaufdiagramm einer Ausführungsform des Verfahrens zum Unterstützen einer neurochirurgischen Operation. Das Verfahren wird beispielsweise mittels einer Anordnung gemäß einer Ausführungsform ausgeführt, wie diese in der Fig. 1 gezeigt ist. Es kann vorgesehen sein, dass ein Nutzer das Verfahren starten muss, beispielsweise wenn der Tumor im Laufe der Operation unter anderem Gewebe freigelegt ist und optisch erfasst werden kann.

In einem Verfahrensschritt 100 wird mindestens eine Abbildung eines Operationsbereichs am Patienten mittels eines medizinischen Visualisierungssystems erfasst.

In einem Verfahrensschritt 101 wird eine kategoriale Lageinformation, die eine anatomische Lage eines im Operationsbereich vorhandenen Tumors beschreibt, erfasst und/oder erhalten.

In einem Verfahrensschritt 102 wird die mindestens eine erfasste Abbildung und die erfasste und/oder erhaltene kategoriale Lageinformation einem trainierten Maschinenlernmodell als Eingangsdaten zugeführt.

In einem Verfahrensschritt 103 wird ein Tumortyp ausgehend von der mindestens einen erfassten Abbildung und der erfassten und/oder erhaltenen kategorialen Lageinformation mittels des trainierten Maschinenlernmodells vorhergesagt, wobei das trainierte Maschinenlernmodell eine den Tumortyp beschreibende Tumortypinformation als Ausgangsdaten bereitstellt.

In einem Verfahrensschritt 104 wird die Tumortypinformation ausgegeben.

Es kann in einem Verfahrensschritt 105 vorgesehen sein, dass ausgehend von dem vorhergesagten Tumortyp mindestens ein Steuersignal erzeugt und/oder bereitgestellt wird, das dazu eingerichtet ist, das medizinische Visualisierungssystem und/oder ein anderes medizinisches Gerät anzusteuern.

Es kann alternativ auch vorgesehen sein, dass das Verfahren kontinuierlich und/oder wiederholt (z.B. im Hintergrund) durchgeführt wird, wobei bei Erreichen eines vorgegebenen Schwellwertes der Klassifikationsgenauigkeit, beispielsweise -90 %, die Tumortypinformation ausgegeben wird.

Weitere Ausführungsformen des Verfahrens wurden bereits mit Bezug auf die Anordnung beschrieben.

### Bezugszeichenliste

- 1: Anordnung
- 2: Datenverarbeitungseinrichtung
- 2-1: Recheneinrichtung
- 2-2: Speicher
- 3: medizinisches Visualisierungssystem
- 3-1: Kamera
- 4: Anzeige- und Bedieneinrichtung
- 5: trainiertes Maschinenlernmodell
- 5-1: Faltungsnetz
- 5-2: Perceptron
- 5-3: Klassifizierungsnetz
- 5-4: erster Embeddingteil
- 5-5: zweiter Embeddingteil
- 5-6: trainiertes erstes Maschinenlernmodell
- 5-7: trainiertes zweites Maschinenlernmodell
- 10: Abbildung
- 11: kategoriale Lageinformation
- 11-1: Angabe zum Hirnlappen
- 11-2: Angabe zur Hirnregion
- 12: Tumortyp
- 12-1: Glioblastom
- 12-2: Metastase
- 13: Tumortypinformation
- 14: visueller Indikator
- 15: Patienteninformation
- 15-1: Geschlecht
- 15-2: Alter
- 15-3: Historie
- 16: Sauger
- 17: Geräuschdaten
- 18: Mikrofon
- 19: haptische Messdaten
- 20: Patient
- 21: Operationsbereich
- 22: Tumor
- 30: Liste (von Tumortypen)
- 40: Steuersignal
- 100-105: Verfahrensschritte

## Patentansprüche

1. Verfahren zum Unterstützen einer neurochirurgischen Operation,
wobei mindestens eine Abbildung (10) eines Operationsbereichs (21) am Patienten (20) mittels eines medizinischen Visualisierungssystems (3) erfasst wird, wobei eine kategoriale Lageinformation (11), die eine anatomische Lage eines im Operationsbereich (21) vorhandenen Tumors (22) beschreibt, erfasst und/oder erhalten wird,
wobei die mindestens eine erfasste Abbildung (10) und die erfasste und/oder erhaltene kategoriale Lageinformation (11) einem trainierten Maschinenlernmodell (5) als Eingangsdaten zugeführt werden,
wobei ein Tumortyp (12) ausgehend von der mindestens einen erfassten Abbildung (10) und der erfassten und/oder erhaltenen kategorialen Lageinformation (11) mittels des trainierten Maschinenlernmodells (5) vorhergesagt wird, wobei das trainierte Maschinenlernmodell (5) eine den Tumortyp (12) beschreibende Tumortypinformation (13) als Ausgangsdaten bereitstellt,
und wobei die Tumortypinformation (13) ausgegeben wird.

2. Verfahren nach Anspruch 1, **dadurch gekennzeichnet, dass** die kategoriale Lageinformation (11) textbasiert erfasst und/oder erhalten wird.

3. Verfahren nach Anspruch 1 oder 2, **dadurch gekennzeichnet, dass** zum Erfassen der kategorialen Lageinformation (11) eine anatomische Karte angezeigt wird, wobei die kategoriale Lageinformation (11) durch Auswahl an der anatomischen Karte erfasst wird.

4. Verfahren nach einem der vorangegangenen Ansprüche, **dadurch gekennzeichnet, dass** die kategoriale Lageinformation (11) eine Information zum Hirnlappen umfasst, der den Tumor beinhaltet.

5. Verfahren nach einem der vorangegangenen Ansprüche, **dadurch gekennzeichnet, dass** die kategoriale Lageinformation (11) eine Information zur Hirnregion umfasst, die den Tumor beinhaltet.

6. Verfahren nach einem der vorangegangenen Ansprüche, **dadurch gekennzeichnet, dass** die kategoriale Lageinformation (11) mindestens eine relationale Information umfasst, die den Tumor in Bezug zu anatomischen Merkmalen verortet.

7. Verfahren nach einem der vorangegangenen Ansprüche, **dadurch gekennzeichnet, dass** zusätzlich mindestens ein visueller Indikator (14) erzeugt und als Bildsignal ausgegeben wird, wobei der visuelle Indikator (14) zumindest einen Bereich in der erfassten mindestens einen Abbildung (10) kennzeichnet, der beim Vorhersagen berücksichtigt wurde.

8. Verfahren nach einem der vorangegangenen Ansprüche, **dadurch gekennzeichnet, dass** mindestens eine Patienteninformation (15) erfasst und/oder erhalten wird, wobei dem trainierten Maschinenlernmodell (5) zusätzlich die mindestens eine erfasste und/oder erhaltene Patienteninformation (15) als Eingangsdaten zugeführt wird, und wobei das trainierte Maschinenlernmodell (15) den Tumortyp (12) zusätzlich unter Berücksichtigung der mindestens einen Patienteninformation (15) vorhersagt.

9. Verfahren nach einem der vorangegangenen Ansprüche, **dadurch gekennzeichnet, dass** das trainierte Maschinenlernmodell (5) ein Faltungsnetz (5-1), ein mehrschichtiges Perceptron (5-2) und ein Klassifizierungsnetz (5-3) aufweist, wobei die erfasste und/oder erhaltene mindestens eine Abbildung (10) mittels des Faltungsnetzes (5-1) in ein ersten Embeddingteil (5-4) überführt wird, wobei die kategoriale Lageinformation (11) und/oder die mindestens eine Patienteninformation (15) mittels des mehrschichtigen Perceptrons (5-2) in einen zweiten Embeddingteil (5-5) überführt wird, und wobei der erste Embeddingteil (5-4) und der zweite Embeddingteil (5-5) dem Klassifikationsnetz (5-3) als Eingangsdaten zugeführt werden.

10. Verfahren nach einem der vorangegangenen Ansprüche, **dadurch gekennzeichnet, dass** Geräuschdaten (17) eines während der neurochirurgischen Operation verwendeten Saugers (16) erfasst und/oder erhalten werden, wobei dem trainierten Maschinenlernmodell (5) zusätzlich die erfassten und/oder erhaltenen Geräuschdaten (17) als Eingangsdaten zugeführt werden, und wobei das trainierte Maschinenlernmodell (5) den Tumortyp (12) zusätzlich unter Berücksichtigung der erfassten und/oder erhaltenen Geräuschdaten (17) vorhersagt.

11. Verfahren nach einem der vorangegangenen Ansprüche, **dadurch gekennzeichnet, dass** eine Elastizität eines Gewebes abbildende haptische Messdaten (19) im Operationsbereich (21) erfasst und/oder erhalten werden, wobei dem trainierten Maschinenlernmodell (5) zusätzlich die erfassten und/oder erhaltenen haptischen Messdaten (19) als Eingangsdaten zugeführt werden, und wobei das trainierte Maschinenlernmodell (5) den Tumortyp (12) zusätzlich unter Berücksichtigung der erfassten und/oder erhaltenen haptischen Messdaten (19) vorhersagt.

12. Verfahren nach einem der vorangegangenen Ansprüche, **dadurch gekennzeichnet, dass** eine Liste (30) von Tumortypen (12) erfasst und/oder vorgegeben wird, wobei dem trainierten Maschinenlernmodell (5) zusätzlich die Liste (30) von Tumortypen (12) als Eingangsdaten zugeführt wird, und wobei das trainierte Maschinenlernmodell (5) den Tumortyp (12) zusätzlich unter Berücksichtigung der Liste (30) von Tumortypen (12) vorhersagt.

13. Verfahren nach einem der vorangegangenen Ansprüche, **dadurch gekennzeichnet, dass** eine Liste (30) von Tumortypen (12) erfasst und/oder vorgegeben wird, wobei für verschiedene Listen (30) von Tumortypen (12) zumindest gruppenweise voneinander verschiedene trainierte Maschinenlernmodelle (5) verwendet werden, wobei das jeweils passende trainierte Maschinenlernmodell (5) ausgehend von der erfassten und/oder vorgegebenen Liste (30) von Tumortypen (12) ausgewählt wird.

14. Verfahren nach einem der vorangegangenen Ansprüche, **dadurch gekennzeichnet, dass** ausgehend von dem vorhergesagten Tumortyp (12) mindestens ein Steuersignal (40) erzeugt und/oder bereitgestellt wird, das dazu eingerichtet ist, das medizinische Visualisierungssystem (3) und/oder ein anderes medizinisches Gerät anzusteuern.

15. Anordnung (1) zum Unterstützen einer neurochirurgischen Operation, umfassend:
eine Datenverarbeitungseinrichtung (2), wobei die
Datenverarbeitungseinrichtung (2) dazu eingerichtet ist,
mindestens eine mittels eines medizinischen Visualisierungssystems (3) erfasste Abbildung (10) eines Operationsbereichs (21) am Patienten (20) zu erhalten,
eine kategoriale Lageinformation (11), die eine anatomische Lage eines im Operationsbereich (21) vorhandenen Tumors (22) beschreibt, zu erhalten,
ein trainiertes Maschinenlernmodell (5) bereitzustellen,
die mindestens eine erfasste Abbildung (10) und die erhaltene kategoriale Lageinformation (11) dem trainierten Maschinenlernmodell (5) als Eingangsdaten zuzuführen,
einen Tumortyp (12) ausgehend von der mindestens einen erfassten Abbildung (10) und der erhaltenen kategorialen Lageinformation (11) mittels des trainierten Maschinenlernmodells (5) vorherzusagen, wobei das trainierte Maschinenlernmodell (5) eine den Tumortyp (12) beschreibende Tumortypinformation (13) als Ausgangsdaten bereitgestellt,
und die Tumortypinformation (13) auszugeben.

16. Anordnung (1) nach Anspruch 15, **dadurch gekennzeichnet, dass** die Anordnung (1) das medizinische Visualisierungssystem (3) umfasst.
